(19) Europäisches Patentamt
European Patent Office
Office européen des brevets

(11) Veröffentlichungsnummer: **0 508 385 A1**

(12) **EUROPÄISCHE PATENTANMELDUNG**

(21) Anmeldenummer: **92106015.8**

(22) Anmeldetag: **07.04.92**

(51) Int. Cl.⁵: **C07C 31/20**, C07C 29/03, B01J 23/36

(30) Priorität: **09.04.91 DE 4111506**

(43) Veröffentlichungstag der Anmeldung: **14.10.92 Patentblatt 92/42**

(84) Benannte Vertragsstaaten: **AT BE CH DE FR GB IT LI NL**

(71) Anmelder: **Peroxid-Chemie GmbH**
**Dr.-Gustav-Adolf-Strasse 3**
**W-8023 Höllriegelskreuth(DE)**

(72) Erfinder: **Warwel, Siegfried, Prof. Dr.**
**Hans-Böckler-Allee 81**
**W-5100 Aachen(DE)**
Erfinder: **Rüsch, Mark, gen. Klaas**
**Reihstrasse 6**
**W-5100 Aachen(DE)**
Erfinder: **Sojka, Michael, Dr.**
**Blümgesgrundstrasse 17**
**W-6460 Gelnhausen(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel, Dr. B. Böhm, Kopernikusstrasse 9, Postfach 86 08 20**
**W-8000 München 86(DE)**

# (54) Verfahren zur Herstellung vicinaler Diole oder/und Epoxide.

(57) Die Erfindung betrifft ein Verfahren zur Herstellung vicinaler Diole oder/und Epoxide durch Oxidation olefinisch ungesättigter Verbindungen mit anorganischen Re(VII)-Verbindungen als Katalysator und $H_2O_2$ als Oxidationsmittel, welches dadurch gekennzeichnet ist, daß man die Reaktion in einem Lösungsmittel, ausgewählt aus der Gruppe, bestehend aus organischen Phosphorsäureestern und Ethern mit einem Siedepunkt von über 50 ° C bei atmosphärischen Bedingungen, durchführt.

EP 0 508 385 A1

Die Erfindung betrifft ein Verfahren zur Herstellung vicinaler Diole oder/und Epoxide durch Oxidation olefinisch ungesättigter Verbindungen mit anorganischen Re(VII)-Verbindungen als Katalysator und $H_2O_2$ als Oxidationsmittel.

Vicinale Diole und Epoxide sind Verbindungen von industrieller Bedeutung. Sie finden Verwendung im Bereich der Wasch- und Reinigungsmittel sowie der Kosmetika und dienen als Kunststoffweichmacher und als Ausgangsstoffe in der Kunststoffherstellung, insbesondere in der Polyurethanchemie.

Die Gewinnung der Epoxide erfolgt in der Technik in der Regel (Ausnahme Ethylenoxid) durch Umsetzung von Olefinen und funktionalisierten Olefinen mit Percarbonsäuren oder Hydroperoxiden. Vicinale Diole werden aus den entsprechenden Epoxiden durch Verseifung hergestellt.

Auch übergangsmetallkatalysierte Oxidationen von C-C-Doppelbindungen mittels $H_2O_2$ sind bekannt (vgl. z.B. R.A. Sheldon, J.K. Kochi: "Metal-Catalyzed Oxidations of Organic Compounds", Academic Press, New York 1981). Das in diesem Zusammenhang klassische Verfahren nutzt dabei das System $OsO_4/H_2O_2$ - ("Milas' Reagenz"), welches Olefine über Osmatester in cis-Glykole überführt:

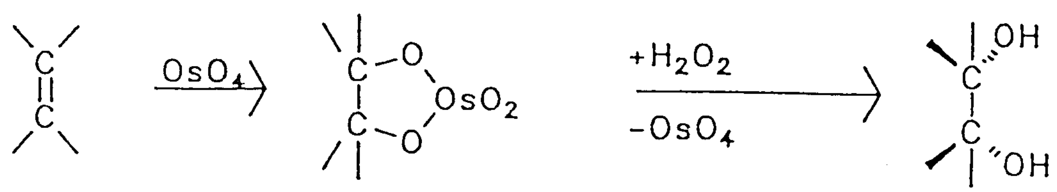


Wegen der starken Giftigkeit von $OsO_4$ und aus Kostengründen fand das Verfahren jedoch keine industrielle Anwendung.

In der DE-OS 39 02 357 A1 werden Epoxidierungen und Hydroxylierungen von Olefinen mittels Katalyse von $CH_3ReO_3$ und anderen metallorganischen Verbindungen des Rheniums beschrieben.

Die Katalysatoren werden dabei durch Umsetzung von $Re_2O_7$ mit hauptgruppenmetallorganischen Verbindungen hergestellt. Dieses ist nachteilig, zumal es sich bei den hauptgruppenmetallorganischen Verbindungen um teure und z.T. stark giftige Substanzen handelt. Das vorzugsweise als Katalysator eingesetzte $CH_3ReO_3$ wird nach Herrmann et al. (Angew.Chem. 100, 420 (1988)) durch Umsetzung von $Re_2O_7$ mit $Sn(CH_3)_4$ gemäß $Re_2O_7 + Sn(CH_3)_4 \rightarrow CH_3\text{-}\overline{ReO_3} + (CH_3)_3Sn\text{-}O\text{-}ReO_3$ hergestellt. Das verwendete $Sn(CH_3)_4$ ist stark giftig und 50 % des eingesetzten $Re_2O_7$ werden in den katalytisch inaktiven Stannylester überführt. Auch ist eine Katalysatorabtrennung und Wiederverwendung nicht möglich, es sei denn, die Rheniumverbindungen werden an polymere Träger fixiert, was die Katalysatorherstellung weiter verkompliziert und verteuert. Darüber hinaus ist die Katalysatoraktivität dann gegenüber den monomeren Rheniumverbindungen wie $CH_3ReO_3$ deutlich reduziert.

Das US-Patent 3,518,285 offenbart ein Verfahren für die Oxidation von Olefinen zu einer oxidierten Verbindung, ausgewählt aus der Klasse, bestehend aus Epoxiden und Ketonen mit derselben Gesamtzahl an Kohlenstoffatomen, umfassend das Kontaktieren eines Kohlenwasserstoffolefins mit 3 bis 20 Kohlenstoffatomen mit einer Rheniumverbindung, ausgewählt aus der Gruppe, bestehend aus Rheniumoxid, Alkalimetall-, Erdalkalimetall- und Ammoniumperrhenaten in einem flüssigen Reaktionsmedium, welches ein Lösungsmittel, das unter Oxidationsbedingungen inert ist, und 3 bis 90 Gew.-% Wasserstoffperoxid enthält, wobei sich das Reaktionsmedium auf einem Druck befindet, der ausreicht, um das Medium als Flüssigkeit zu halten, und bei einer Temperatur von -50 °C bis 150 °C, die ausreicht, um das Olefin zur oxidierten Verbindung zu oxidieren. Als Beispiele für geeignete Lösungsmittel werden genannt: aromatische Kohlenwasserstoffe, aliphatische Kohlenwasserstoffe, halogenierte Kohlenwasserstoffe, Carbonsäureester und Nitrile. Weiterhin wird das während der Reaktion erzeugte Wasser aus dem Reaktionsmedium entfernt, um die Oxidation des Olefins aufrechtzuerhalten. Dieses Verfahren hat jedoch insofern schwerwiegende Nachteile, daß nur sehr geringe Ausbeuten an Diolen und Epoxiden aus den olefinisch ungesättigten Verbindungen erhalten werden. Hingegen wird die Bildung von weiteroxidierten Folgeprodukten (Ketone, Carbonsäuren) oder polymeren Kondensationsprodukten beobachtet.

Aufgabe der vorliegenden Erfindung war es somit, ein Verfahren zur Herstellung von Diolen oder/und Epoxiden durch Oxidation von olefinisch ungesättigten Verbindungen zu entwickeln, bei dem die Nachteile des Standes der Technik zumindest teilweise beseitigt werden.

Insbesondere sollte ein Verfahren entwickelt werden, das unter Verwendung von ungiftigen und wiederverwendbaren Katalysatoren Epoxide und vicinale Diole in hoher Ausbeute liefert.

Die Lösung der erfindungsgemäßen Aufgabe gelingt durch ein Verfahren zur Herstellung vicinaler Diole oder/und Epoxide durch Oxidation olefinisch ungesättigter Verbindungen mit anorganischen Re(VII)-Verbin-

dungen als Katalysator und $H_2O_2$ als Oxidationsmittel, welches dadurch gekennzeichnet ist, daß man die Reaktion in einem Lösungsmittel, ausgewählt aus der Gruppe, bestehend aus organischen Phosphorsäureestern und Ethern mit einem Siedepunkt von über 50 °C bei atmosphärischen Bedingungen, durchführt.

Die sehr gute Eignung von anorganischen Re(VII)-Verbindungen als Katalysatoren für die Oxidation von Olefinen bei Verwendung bestimmter Lösungsmittel ist in Anbetracht des Standes der Technik sehr überraschend. So wird in der DE-OS 39 02 357, in welcher der Einsatz metallorganischer Rheniumverbindungen beschrieben wird, darauf hingewiesen, daß der Einsatz von Rheniumoxiden wie z.B. $Re_2O_7$ oder Perrhenaten wie z.B. $Na(ReO_4)$ als Katalysator für die Oxidation von Olefinen erfolglos war.

Für das erfindungsgemäße Verfahren ist die Verwendung von geeigneten Lösungsmitteln wesentlich. Es wurde festgestellt, daß organische Phosphorsäureester und Ether mit einem Siedepunkt von über 50 °C bei atmosphärischen Bedingungen gute Ausbeuten liefern. Dagegen wurde gefunden, daß die im US-Patent 3,518,285 offenbarten Lösungsmittel, wie etwa aromatische Kohlenwasserstoffe, aliphatische Kohlenwasserstoffe, halogenierte Kohlenwasserstoffe, Ester und Nitrile keine akzeptablen Ausbeuten erbringen.

Verwendet man einen organischen Phosphorsäureester als Lösungsmittel, so sind Phosphorsäuretrialkylester bevorzugt, bei denen jeder Alkylrest 1 bis 10 C-Atome aufweisen kann und wobei die Alkylreste gleich und verschieden sein können. Besonders bevorzugt sind Phosphorsäuretrialkylester, bei denen jeder Alkylrest 2 bis 8 C-Atome aufweist. Am meisten bevorzugt sind Triethylphosphat und Tri-n-butylphosphat.

Bei Verwendung eines Ethers mit einem Siedepunkt von über 50 °C bei atmosphärischen Bedingungen sind cyclische oder acyclische Ether mit mehreren, insbesondere 2 bis 4 Ethergruppen bevorzugt. Bevorzugte Beispiele solcher Verbindungen sind 1,4-Dioxan, Dimethoxyethan (Glyme) und Diethylenglykoldimethylether (Diglyme). Die Siedepunkte für 1,4-Dioxan, Glyme und Diglyme sind bei 101 °C, 84 °C bzw. 160 °C. Es können jedoch auch hochsiedende Ether mit nur einer Ethergruppe erwendet werden, wie etwa Di-n-butylether (Siedepunkt 140 °C) und Dibenzylether (Siedepunkt 300 °C).

Als Ausgangsverbindung für das erfindungsgemäße Verfahren werden olefinisch ungesättigte Verbindungen, d.h. cyclische oder acyclische Verbindungen mit einer oder mehreren C=C-Doppelbindungen, d.h. Monoolefine, Diolefine oder Polyene eingesetzt. Bei acyclischen Olefinen können C=C-Doppelbindungen sowohl endständig sein als auch innerhalb des Moleküls liegen. Diese Verbindungen können gegebenenfalls mit einer oder mehreren funktionellen Gruppen wie etwa einer Hydroxylgruppe, einer Carbonsäuregruppe, einer Carbonsäureestergruppe oder einer Arylgruppe (insbesondere einer Phenylgruppe) substituiert sein. Beispiele für geeignete offenkettige unsubstituierte Olefine sind etwa Octene (z.B. 1-Octen), Decene (z.B. 5-Decen), Dodecene (z.B. 1-Dodecen oder 6-Dodecen), Tetradecene (z.B. 1-Tetradecen oder 7-Tetradecen) sowie Hexadecene und Octadecene. Beispiele für cyclische Olefine sind etwa Cyclohexen, Cyclohepten, Cyclooocten oder Cyclodecen. Beispiele für substituierte Olefine sind etwa offenkettige ungesättigte Carbonsäuren (z.B. 10-Undecensäure) sowie deren Alkyl- oder Arylester (z.B. 10-Undecensäuremethylester).

Als Katalysator werden anorganische Re(VII)-Verbindungen verwendet. Beispiele für solche Verbindungen sind $Re_2O_7$ oder Perrhenate der allgemeinen Formel $M(ReO_4)$ oder $M'(ReO_4)_2$, wobei M ein Alkalimetall oder Ammonium und M' ein Erdalkalimetall bedeutet. Die Rheniumverbindungen können dabei auch in Form von Verbindungen mit niedriger Oxidationsstufe (einschließlich als metallisches Rhenium oder als Rhenium-Carbonyl-Komplex) zum Reaktionsgemisch zugegeben werden. Derartige Verbindungen sind ebenfalls katalytisch wirksam, da sie durch das im Reaktionsgemisch vorhandene $H_2O_2$ zu katalytisch wirksamen Rhenium(VII)-Verbindungen oxidiert werden (siehe z.B. US-Patent 3,518,285).

Das erfindungsgemäße Verfahren wird bei einem sauren pH-Wert, vorzugsweise bei einem pH-Wert von weniger als 3 durchgeführt. Besonders bevorzugt ist ein pH-Wert zwischen 0 und 2. Als am günstigsten hat sich ein pH-Wert von etwa 1 erwiesen. Bei Verwendung von $Re_2O_7$ als Katalysator stellt sich (etwas abhängig vom Lösungsmittel) ohne weitere Maßnahmen ein optimaler pH-Wert von ca. 1 ein. Verwendet man Perrhenate als Katalysator, so wird günstigerweise der pH-Wert des Reaktionsgemisches durch Zusatz einer starken anorganischen oder organischen Säure auf einen Wert von weniger als 3, vorzugsweise auf etwa 1 eingestellt. Die dafür geeigneten anorganischen und organischen Säuren sollten unter den Reaktionsbedingungen nicht durch $H_2O_2$ oxidierbar sein. Beispiele für anorganische Säuren sind $H_2SO_4$, $H_3PO_4$ und $HClO_4$, wobei $HClO_4$ bevorzugt ist. Beispiele für organische Säuren sind Alkyl- oder Arylsulfonsäuren wie etwa Toluolsulfonsäure.

Der Katalysator wird für das erfindungsgemäße Verfahren vorzugsweise in Konzentrationen von 0,2 bis 5 mol-%, bezogen auf eine oxidierbare C=C-Doppelbindung der olefinisch ungesättigten Ausgangsverbindung eingesetzt. Besonders bevorzugt ist die Verwendung des Katalysators in Konzentrationen von 0,8 bis 1,2 mol-%, bezogen auf eine oxidierbare C=C-Doppelbindung der olefinisch ungesättigten Ausgangsverbindung.

Die für das erfindungsgemäße Verfahren verwendeten Katalysatoren zeichnen sich insbesondere

dadurch aus, daß sie nach Abtrennung der Reaktionsprodukte aus dem Reaktionsgemisch (z.B. durch Filtration) im Reaktionsmedium verbleiben und in dieser Form erneut als Katalysatorlösung verwendet werden können, ohne daß die katalytische Aktivität abfällt oder irgend eine Katalysatoraufbereitung oder -aktivierung erfolgen muß. Auf diese Weise ist eine vielfache Wiederverwertung des Katalysators möglich.

Wasserstoffperoxid wird bei dem erfindungsgemäßen Verfahren in Konzentrationen von 30 bis 85 %, vorzugsweise als 60 %ige wäßrige Lösung eingesetzt. Das molare Verhältnis von $H_2O_2$ zu einer oxidierbaren C=C-Doppelbindung der olefinisch ungesättigten Ausgangsverbindung beträgt vorzugsweise von 1:1 bis 4:1, besonders bevorzugt etwa 1,2:1. Es ist jedoch auch der Einsatz von Olefin im Überschuß möglich.

Die Temperaturen bei dem erfindungsgemäßen Verfahren liegen in der Regel zwischen Raumtemperatur und 100°C, vorzugsweise zwischen 70°C und 90°C. In speziellen Fällen können jedoch auch Temperaturen unterhalb 20°C gewählt werden.

Als Reaktionsprodukte bei der Oxidation olefinisch ungesättigter Verbindungen werden überwiegend vicinale Diole erhalten. Durch die Wahl tieferer Reaktionstemperaturen und höherer Katalysatorkonzentrationen können jedoch auch solche Bedingungen eingestellt werden, unter denen Epoxide das Hauptprodukt der Reaktion darstellen.

Die Durchführung der Reaktion geschieht vorzugsweise so, daß die zu oxidierende Verbindung zum Lösungsmittel gegeben und dann der Katalysator hinzugefügt wird. Anschließend erfolgt das Zutropfen von $H_2O_2$. Dies kann bei Raumtemperatur geschehen (wie in den Beispielen), so daß das Reaktionsgemisch erst nach Zugabe von $H_2O_2$ auf die Reaktionstemperatur aufgeheizt wird. Nach Beendigung der Reaktion wird das Reaktionsgemisch vorzugsweise auf 0°C abgekühlt und mit einem milden Reduktionsmittel (z.B. $Na_2SO_3$) in wäßriger Lösung versetzt. Die Reaktionsprodukte können aus dem resultierenden Gemisch beispielsweise durch Extraktion mit organischen Lösungsmitteln oder - falls sie unlöslich sind - durch Filtration entfernt werden. Es werden jedoch gleiche Ergebnisse erhalten, wenn das Gemisch bereits vor der Zugabe von $H_2O_2$ auf Reaktionstemperatur aufgeheizt wird.

Die Erfindung wird durch die nachfolgenden Beispiele näher erläutert.

## Beispiel 1

Oxidation von 1-Octen

0,05 mol 1-Octen (5,6 g) werden in 20 ml Triethylphosphat gelöst und 0,5 mmol $Re_2O_7$ (0,24 g) hinzugefügt. Dazu wird unter Rühren bei Raumtemperatur langsam 0,06 mol $H_2O_2$ (3,4 g, 60 %) zugetropft. Nach 16 h bei 90°C wird das Reaktionsgemisch auf 0°C abgekühlt und mit ca. 30 ml gesättigter Natriumsulfitlösung versetzt. Die Lösung wird mehrfach mit Ether extrahiert und die vereinigten organischen Extrakte werden am Rotationsverdampfer eingeengt.

Das verbleibende Produktgemisch wird einer GC- und GC/MS-Analyse unterworfen. Aufgrund von authentischen Vergleichssubstanzen wird festgestellt, daß es neben nicht umgesetztem Edukt 1,2-Octandiol und 1-Octenoxid enthält. Durch Zusatz eines GC-Standards (Oenanthsäureethylester) werden unter Berücksichtigung vorher mittels reiner Substanzen ermittelter Korrekturfaktoren die Mengen der gebildeten Reaktionsprodukte quantitativ bestimmt. Die Ausbeute bezieht sich in diesem und in den folgenden Beispielen jeweils auf das eingesetzte Olefin.
Ausbeute an 1,2-Octandiol: 37 % d. Th. bez. auf Olefin
Ausbeute an 1-Octenoxid: 11 % d. Th. bez. auf Olefin
Bei Verwendung von Tri-n-butylphosphat als Lösungsmittel und ansonsten gleicher Versuchsdurchführung wurden 41 % Diol und 9 % Epoxid erhalten. Mit Triisooctylphosphat wurden 27 % Diol und 8 % Epoxid erhalten.

## Beispiel 2

Oxidation von 7-Tetradecen

0,03 mol 7-Tetradecen (5,9 g) werden in 20 ml Triethylphosphat gelöst und 0,3 mmol $Re_2O_7$ (0,14 g) hinzugefügt. Dazu wird unter Rühren langsam 0,036 mol $H_2O_2$ (2,0 g, 60 %) zugetropft. Anschließend wird 16 h bei 90°C gerührt.

Die Aufarbeitung erfolgt nach zwei Verfahrensweisen:

a) Man versetzt das Reaktionsgemisch mit dem doppelten Volumen Ethanol (ca. 60 ml) und anschließend mit ca. 20 ml wäßriger Natriumsulfitlösung. Die Lösung wird mehrfach mit Ether extrahiert, die vereinigten organischen Extrakte werden am Rotationsverdampfer eingeengt.

Das verbleibende Produktgemisch wird zwecks Überführung gebildeter Diole in ihre Trimethylsilylether in üblicher Weise silyliert und anschließend einer GC- und GC/MS-Analyse unterworfen. Aufgrund von authentischen Vergleichssubstanzen wird festgestellt, daß es neben nicht umgesetztem Edukt 7,8-Tetradecandiol (in Form der Trimethylsilylether) enthält. Durch Zusatz eines GC-Standards (Phthalsäurediethylester) werden unter Berücksichtigung vorher mittels reiner Substanzen ermittelter Korrekturfaktoren die Mengen der gebildeten Trimethylsilylether des 7,8-Tetradecandiols quantitativ bestimmt. Ausbeute an silyliertem 7,8-Tetradecandiol: 67 % d. Th. bez. auf Olefin

b) Man kühlt auf 0 °C ab und versetzt mit ca. 20 ml wäßriger Natriumsulfitlösung. Dabei fällt ein weißer Feststoff aus, der durch Filtration abgetrennt wird.

Durch Elementaranalyse und Vergleich des GC/MS-Spektrums mit einer authentischen Probe wird der Feststoff als 7,8-Tetradecandiol identifiziert.

Smp.: 70 - 73 °C, Smp. einer authentischen Probe: 69 - 70 °C Ausbeute an 7,8-Tetradecandiol: 4,1 g = 59 % d. Th. bez. auf Olefin

Beispiel 3

Oxidation von 1-Tetradecen

0,03 mol 1-Tetradecen (5,9 g) werden in 20 ml 1,4-Dioxan gelöst und 0,3 mmol $Re_2O_7$ (0,14 g) hinzugefügt. Dazu wird unter Rühren langsam 0,036 mol $H_2O_2$ (2,0 g, 60 %) zugetropft. Anschließend wird 16 h bei 90 °C gerührt. Man kühl auf 0 °C ab und versetzt mit ca. 20 ml wäßriger Natriumsulfitlösung. Dabei fällt ein weißer Feststoff aus, der durch Filtration abgetrennt wird.

Durch Elementaranalyse und Vergleich des GC/MS-Spektrums mit einer Probe, die durch Hydroxylierung von 1-Tetradecen mit Perameisensäure hergestellt wird, wird der Feststoff als 1,2-Tetradecandiol identifiziert.

Smp.: 59 - 61 °C, Smp. einer authentischen Probe: 62 - 64 °C Ausbeute an 1,2-Tetradecandiol: 4,2 g = 61 % d. Th. bez. auf Olefin

Beispiel 4

Oxidation von 7-Tetradecen

0,03 mol 7-Tetradecen (5,9 g) werden in 20 ml 1,4-Dioxan gelöst und 0,3 mmol $Re_2O_7$ (0,14 g) hinzugefügt. Dazu wird unter Rühren langsam 0,036 mol $H_2O_2$ (2,0 g, 60 %) zugetropft. Anschließend wird 16 h bei 90 °C gerührt. Man kühlt auf 0 °C ab und versetzt mit ca. 20 ml wäßriger Natriumsulfitlösung. Dabei fällt ein weißer Feststoff aus, der durch Filtration abgetrennt wird.

Aubeute an 7,8-Tetradecandiol: 4,9 g = 71 % d. Th. bez. auf Olefin.

Beispiel 4a

Arbeitet man nach Beispiel 4 und verwendet statt $Re_2O_7$ 0,20 g Dirheniumdecacarbonyl, so erhält man 4,6 g Diol (67 % d. Th. bez. auf Olefin).

Beispiel 5

Oxidation von Cyclohexen

0,05 mol Cyclohexen (4,1 g) werden in 20 ml Triethylphosphat gelöst und 0,5 mmol (0,24 g) $Re_2O_7$ hinzugefügt. Dazu wird unter Rühren langsam 0,06 mol $H_2O_2$ (3,4 g, 60 %) zugetropft. Anschließend wird 16 h bei 70 °C gerührt. Danach kühlt man auf 0 °C ab und versetzt mit ca. 20 ml wäßriger gesättigter Natriumsulfitlösung. Das Reaktionsgemisch wird mehrfach mit Ether extrahiert und das Reaktionsprodukt durch fraktionierte Destillation abgetrennt.

Das Produkt wird durch Vergleich des GC/MS-Spektrums mit dem einer authentischen Probe und durch Elementaranalyse identifiziert.

Ausbeute an 1,2-Cyclohexandiol: 4,3 g = 74 % d. Th. bez. auf Olefin

Beispiel 6

Oxidation weiterer Olefine

Unter Reaktionsbedingungen analog zu Beispiel 3 wurden des weiteren folgende Olefine umgesetzt:

| | Olefin | Reaktionsprodukt | Ausbeute % d. Th.bez. auf Olefin |
|---|---|---|---|
| a) | 1-Dodecen | 1,2-Dodecandiol | 62 |
| b) | 1-Hexadecen | 1,2-Hexadecandiol | 80 |
| c) | 1-Octadecen | 1,2-Octadecandiol | 77 |
| d) | 4-Octen | 4,5-Octandiol | 60 |
| e) | 5-Decen | 5,6-Decandiol | 52 |
| f) | 6-Dodecen | 6,7-Dodecandiol | 54 |
| g) | 9-Octandecen | 9,10-Octdecandiol | 80 |
| h) | Cyclohepten | 1,2-Cycloheptandiol | 35 |
| i) | Cycloocten | 1,2-Cyclooctandiol | 52 |
| k) | Cyclododecen | 1,2-Cyclododecandiol | 68 |

Beispiel 7

Oxidation von 9-Octadecen mit Katalysatorrecycling

a) 0,03 mol 9-Octadecen (7,6 g) werden in 20 ml 1,4-Dioxan gelöst und 0,3 mmol $Re_2O_7$ (0,14 g) hinzugefügt. Dazu wird unter Rühren langsam 0,036 mol $H_2O_2$ (2,0 g, 60 %) zugetropft. Anschließend wird 16 h bei 90 ° C gerührt. Danach kühlt man auf 0 ° C ab. Dabei fällt ein weißer Feststoff aus, der durch Filtration abgetrennt wird.

Durch Elementaranalyse und Vergleich des GC/MS-Spektrums mit einer authentischen Probe wird der Feststoff als 9,10-Octadecandiol identifiziert.

Ausbeute an 9,10-Octadecandiol: 4,4 g = 51 % d. Th. bez. auf Olefin

b) Das Filtrat von Versuch 7a) wird in das Reaktionsgefäß zurückgeführt, erneut 0,03 mol 9-Octadecen (7,6 g) sowie 5 ml 1,4-Dioxan zugegeben. Man tropft weiter 0,036 mol $H_2O_2$(2,0 g, 60 %) zu, rührt wiederum 16 h bei 90 ° C, kühlt ab und isoliert das Produkt durch Filtration.

Ausbeute an 9,10-Octadecandiol: 6,7 g = 78 % d. Th. bez. auf Olefin

c) Das Filtrat von Versuch 7b) wird in das Reaktionsgefäß zurückgeführt, erneut 0,03 mol 9-Octadecen (7,6 g) sowie 5 ml 1,4-Dioxan zugegeben. Man tropft weitere 0,036 mol $H_2O_2$ (2,0 g, 60 %) zu, rührt wiederum 16 h bei 90 ° C, kühlt ab und isoliert das Produkt durch Filtration.

Ausbeute an 9,10-Octadecandiol: 7,0 g = 81 % d. Th. bez. auf Olefin

d) Das Filtrat von Versuch 7c) wird in das Reaktionsgefäß zurückgeführt, erneut 0,03 mol 9-Octadecen (7,6 g) sowie 5 ml 1,4-Dioxan zugegeben. Man tropft weitere 0,036 mol $H_2O_2$ (2,0 g, 60 %) zu, rührt wiederum 16 h bei 90 ° C, kühlt ab und isoliert das Produkt durch Filtration.

Ausbeute an 9,10-Octadecandiol: 6,1 g = 71 % d. Th. bez. auf Olefin

e) Das Filtrat von Versuch 7d) wird in das Rekationsgefäß zurückgeführt, erneut 0,03 mol 9-Octadecen (7,6 g) sowie 5 ml 1,4-Dioxan zugegeben. Man tropft weitere 0,036 mol $H_2O_2$ (2,0 g, 60 %) zu, rührt wiederum 16 h bei 90 ° C, kühlt ab und isoliert das Produkt durch Filtration.

Ausbeute an 9,10-Octadecandiol: 5,7 g = 66 % d. Th. bez. auf Olefin

f) Das Filtrat von Versuch 7e) wird in das Reaktionsgefäß zurückgeführt, erneut 0,03 mol 9-Octadecen (7,6 g) sowie 5 ml 1,4-Dioxan zugegeben. Man tropft weitere 0,036 mol $H_2O_2$ (2,0 g, 60 %) zu, rührt wiederum 16 h bei 90 ° C, kühlt ab und isoliert das Produkt durch Filtration.

Aubeute an 9,10-Octadecandiol: 7,8 g = 91 % d. Th. bez. auf Olefin

Die Gesamtausbeute an 9,10-Octadecandiol beträgt bei sechsmaligem Einsatz des Katalysators 73 % d. Th. bez. auf das insgesamt eingesetzte Olefin.

Beispiel 8

Oxidation von 7-Tetradecen

0,03 mol 7-Tetradecen (5,9 g) werden in 20 ml 1,4-Dioxan gelöst und 0,3 mmol $KReO_4$ (0,09 g)

hinzugefügt. Man fügt soviel $HClO_4$ (60 %ige wäßrige Lösung) hinzu, daß der pH-Wert auf 1 absinkt. Dazu wird unter Rühren langsam 0,036 mol $H_2O_2$ (2,0 g, 60 %) zugetropft. Anschließend wird 16 h bei 90°C gerührt. Danach kühlt man auf 0°C ab und versetzt mit ca. 20 ml wäßriger gesättigter Natriumsulfitlösung. Dabei fällt ein weißer Feststoff aus, der durch Filtration abgetrennt wird.
Ausbeute an 7,8-Tetradecandiol: 3,9 g = 56 % d. Th. bez. auf Olefin

Beispiel 9

Oxidation von 10-Undecensäuremethylester

0,03 mol 10-Undecensäuremethylester (6,0 g) werden in 20 ml 1,4-Dioxan gelöst und 0,3 mmol $Re_2O_7$ (0,14 g) hinzugefügt. Dazu wird unter Rühren langsam 0,036 mol $H_2O_2$ (2,0 g, 60 %) zugetropft. Anschließend wird 16 bei 90°C gerührt. Danach kühlt man auf 0°C ab und versetzt mit ca. 20 ml wäßriger, gesättigter Natriumsulfitlösung. Das Reaktionsgemisch wird mit Essigester extrahiert, und die vereinigten organischen Extrakte werden am Rotationsverdampfer eingeengt.

Das verbleibende Produktgemisch wird einer GC- und GC/MS-Analyse unterworfen. Aufgrund von authentischen Vergleichssubstanzen, hergestellt durch Epoxidierung von 10-Undecensäuremethylester mit Peressigsäure bzw. durch Hydroxylierung desselben mit Perameisensäure wird festgestellt, daß es neben nicht umgesetztem Edukt 10,11-Dihydroxyundecansäuremethylester und 10-Epoxyundecensäuremethylester enthält. Durch Zusatz eines GC-Standards (Phthalsäurediethylester) werden unter Berücksichtigung vorher mittels reiner Substanzen ermittelter Korrekturfaktoren die Mengen der gebildeten Reaktionsprodukte quantitativ bestimmt.
Ausbeute an 10,11-Dihydroxyundecansäuremethylester:
33 % d. Th. bez. auf 10-Undecensäuremethylester
Ausbeute an 10-Epoxyundecensäuremethylester:
24 % d. Th. bez. auf 10-Undecensäuremethylester

Beispiel 10

Oxidation von 7-Tetradecen in Glyme (1,2-Dimethoxyethan)

0,03 mol 7-Tetradecen (5,9 g) werden in 20 ml Glyme (Dimethoxyethan) gelöst und 0,3 mmol $Re_2O_7$ - (0,14 g) hinzugefügt. Dazu wird unter Rühren langsam 0,036 mol $H_2O_2$ (2,0 g, 60 %) zugetropft. Anschließend wird 16 h bei 90°C gerührt. Man kühlt auf 0°C ab und versetzt mit ca. 20 ml wäßriger, gesättigter Natriumsulfitlösung. Dabei fällt ein weißer Feststoff aus, der durch Filtration abgetrennt wird.
Ausbeute an 7,8-Tetradecandiol: 4,5 g = 65 % d. Th. bez. auf Olefin

Beispiel 11

Vergleichsbeispiel (entspricht Beispiel 1 der US-PS 3,518,285):

Oxidation von 1-Octen in Toluol als Lösungsmittel unter Wasserentzug durch Azeotropdestillation:
0,10 mol 1-Octen (11,2 g) werden in 40 ml Toluol gelöst und 1 mmol $Re_2O_7$ (0,48 g) hinzugefügt. Dazu wird unter Rühren langsam 0,12 mol $H_2O_2$ (6,8 g, 60 %) zugetropft. Über einen Wasserabscheider wird während der Versuchsdauer von 16 h kontinuierlich das entstehende Wasser abgetrennt.

Anschließend wird das Reaktionsgemisch auf 0°C abgekühlt und mit ca. 50 ml gesättigter Natriumsulfitlösung versetzt. Die Lösung wird mehrfach mit Ether extrahiert und die vereinigten organischen Extrakte werden am Rotationsverdampfer eingeengt.
Ausbeute an 1,2-Octandiol: 4 % d. Th. bez. auf Olefin
Ausbeute an 1-Octenoxid: 2 % d. Th. bez. auf Olefin

Beispiel 12

Vergleichsbeispiel zu den in der US-PS 3,518,285 vorgeschlagenen Lösungsmitteln; Oxidation von 1-Octen

0,05 mol 1-Octen (5,6 g) werden in 20 ml des entsprechenden Lösungsmittels gelöst - oder falls dies nicht möglich ist, suspendiert - und 0,5 mmol $Re_2O_7$ (0,24 g) hinzugefügt. Dazu wird unter Rühren langsam 0,06 mol $H_2O_2$ (3,4 g, 60 %) zugetropft. Nach 16 h bei 90°C - bzw. beim Siedepunkt des entsprechenden

Lösungsmittels, falls dieser tiefer liegt - wird das Reaktionsgemisch auf 0°C abgekühlt und mit ca. 30 ml gesättigter Natriumsulfitlösung versetzt. Die Lösung wird mehrfach mit Ether extrahiert und die vereinigten organischen Extrakte werden am Rotationsverdampfer eingeengt.

a) n-Hexan als Lösungsmittel
Ausbeute an 1,2-Octandiol: 0 % d. Th. bez. auf Olefin
Ausbeute an 1-Octenoxid: 0 % d. Th. bez. auf Olefin
b) Toluol als Lösungsmittel
Ausbeute an 1,2-Octandiol: 14 % d. Th. bez. auf Olefin
Ausbeute an 1-Octenoxid: 1 % d. Th. bez. auf Olefin
c) 1,2-Dichlorethan als Lösungsmittel
Ausbeute an 1,2-Octandiol: 18 % d. Th. bez. auf Olefin
Ausbeute an 1-Octenoxid: 4 % d. Th. bez. auf Olefin
d) Diethylcarbonat als Lösungsmittel
Ausbeute an 1,2-Octandiol: 15 % d. Th. bez. auf Olefin
Ausbeute an 1-Octenoxid: 0 % d. Th. bez. auf Olefin
e) Acetonitril als Lösungsmittel
Ausbeute an 1,2-Octandiol: 6 % d. Th. bez. auf Olefin
Ausbeute an 1-Octenoxid: 2 % d. Th. bez. auf Olefin

Damit ist die Überlegenheit der erfindungsgemäßen Lösungsmittel über die in der US-PS 3,518,285 genannten Lösungsmittel evident.

**Patentansprüche**

**1.** Verfahren zur Herstellung vicinaler Diole oder/und Epoxide durch Oxidation olefinisch ungesättigter Verbindungen mit anorganischen Re(VII)-Verbindungen als Katalysator und $H_2O_2$ als Oxidationsmittel,
**dadurch gekennzeichnet,**
daß man die Reaktion in einem Lösungsmittel, ausgewählt aus der Gruppe, bestehend aus organischen Phosphorsäureestern und Ethern mit einem Siedepunkt von über 50°C bei atmosphärischen Bedingungen, durchführt.

**2.** Verfahren nach Anspruch 1,
**dadurch gekennzeichnet,**
daß man den Katalysator in Konzentrationen von 0,2 bis 5 mol-%, bezogen auf eine oxidierbare C=C-Doppelbindung der olefinisch ungesättigten Verbindung, einsetzt.

**3.** Verfahren nach Anspruch 2,
**dadurch gekennzeichnet,**
daß man den Katalysator in Konzentrationen von 0,8 bis 1,2 mol-%, bezogen auf eine oxidierbare C=C-Doppelbindung der olefinisch ungesättigten Verbindung, einsetzt.

**4.** Verfahren nach einem der vorhergehenden Ansprüche,
**dadurch gekennzeichnet,**
daß man die Reaktion bei einem pH-Wert von weniger als 3, insbesondere zwischen 0 und 2 durchführt.

**5.** Verfahren nach einem der vorhergehenden Ansprüche,
**dadurch gekennzeichnet,**
daß man $Re_2O_7$ als Katalysator verwendet.

**6.** Verfahren nach einem der Ansprüche 1 bis 4,
**dadurch gekennzeichnet,**
daß man ein Perrhenat der allgemeinen Formeln $M[ReO_4]$ oder $M'[ReO_4]_2$ verwendet, wobei M ein Alkalimetall oder Ammonium und M' ein Erdalkalimetall bedeutet.

**7.** Verfahren nach Anspruch 6,
**dadurch gekennzeichnet,**
daß man den pH-Wert des Reaktionsgemisches durch Zusatz einer unter den Reaktionsbedingungen nicht oxidierbaren anorganischen oder organischen Säure auf einen Wert von weniger als 3 einstellt.

**8.** Verfahren nach Anspruch 7,
**dadurch gekennzeichnet,**
daß man als anorganische Säure $HClO_4$, $H_3PO_4$ oder $H_2SO_4$ und als organische Säure eine Alkyl- oder Arylsulfonsäure verwendet.

**9.** Verfahren nach einem der Ansprüche 1 bis 4,
**dadurch gekennzeichnet,**
daß man Rhenium oder Rheniumverbindungen mit niederer Oxidationsstufe zum Reaktionsgemisch zugibt, wobei diese Substanzen durch $H_2O_2$ zu katalytisch wirksamen Rhenium(VII)-Verbindungen oxidiert werden.

**10.** Verfahren nach Anspruch 9,
**dadurch gekennzeichnet,**
daß man metallisches Rhenium oder Dirheniumdecacarbonyl zugibt.

**11.** Verfahren nach einem der Ansprüche 1 bis 10,
**dadurch gekennzeichnet,**
daß man als Lösungsmittel einen Phosphorsäuretrialkylester verwendet, wobei jeder Alkylrest 1 bis 10 C-Atome aufweisen kann.

**12.** Verfahren nach Anspruch 11,
**dadurch gekennzeichnet,**
daß man als Lösungsmittel einen Phosphorsäuretrialkylester verwendet, wobei jeder Alkylrest 2 bis 8 C-Atome aufweisen kann.

**13.** Verfahren nach Anspruch 12,
**dadurch gekennzeichnet,**
daß man als Lösungsmittel Triethylphosphat oder Tri-n-butylphosphat verwendet.

**14.** Verfahren nach einem der Ansprüche 1 bis 10,
**dadurch gekennzeichnet,**
daß man als Lösungsmittel einen cyclischen oder acyclischen Ether mit mehreren, insbesondere 2 bis 4 Ethergruppen verwendet.

**15.** Verfahren nach Anspruch 14,
**dadurch gekennzeichnet,**
daß man als Lösungsmittel 1,4-Dioxan verwendet.

**16.** Verfahren nach Anspruch 13,
**dadurch gekennzeichnet,**
daß man als Lösungsmittel Glyme oder Diglyme verwendet.

**17.** Verfahren nach einem der vorhergehenden Ansprüche,
**dadurch gekennzeichnet,**
daß man aus dem Reaktionsgemisch die Reaktionsprodukte abtrennt und den Rückstand erneut als Katalysatorlösung in die Reaktion einsetzt.

**18.** Verfahren nach Anspruch 17,
**dadurch gekennzeichnet,**
daß die Abtrennung der Reaktionsprodukte durch Filtration erfolgt.

**19.** Verfahren nach einem der vorhergehenden Ansprüche,
**dadurch gekennzeichnet,**
daß man als olefinische ungesättigte Verbindungen cyclische oder acyclische Monoolefine, Diolefine oder Polyene verwendet, die gegebenenfalls mit einer oder mehreren funktionellen Gruppen, ausgewählt aus Hydroxyl-, Carbonsäure-, Carbonsäureester- oder Arylgruppen, substituiert sein können.

**20.** Verfahren nach einem der vorhergehenden Ansprüche,

**dadurch gekennzeichnet,**
daß man $H_2O_2$ in einem molaren Verhältnis von 1:1 bis 4:1 zu einer oxidierbaren C=C-Doppelbindung der olefinisch ungesättigten Verbindung einsetzt.

**21.** Verfahren nach Anspruch 20,
**dadurch gekennzeichnet,**
daß man $H_2O_2$ in einem molaren Verhältnis von etwa 1,2:1 zu einer oxidierbaren C=C-Doppelbindung der olefinisch ungesättigten Verbindung einsetzt.

**22.** Verfahren nach einem der vorhergehenden Ansprüche,
**dadurch gekennzeichnet,**
daß man die Reaktion bei einer Temperatur von Raumtemperatur bis 100 °C durchführt.

**23.** Verfahren nach Anspruch 22,
**dadurch gekennzeichnet,**
daß man die Reaktion bei einer Temperatur von 70 °C bis 90 °C durchführt.

Europäisches Patentamt

# EUROPÄISCHER RECHERCHENBERICHT

Nummer der Anmeldung

EP 92 10 6015

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | CHEMICAL ABSTRACTS, vol. 116, no. 7, 17. Februar 1992, Columbus, Ohio, US; abstract no. 58690E, S. WARWEL ET AL.: 'Formation of vicinal diols by Re2O7-catalysed hydroxylation of alkenes with hydrogen peroxide.' Seite 799 ; Spalte 2 ; & J. Chem. Soc. Chem Commun. 1991, (21), 1578-9 * Zusammenfassung * | 1-3,5, 14,15, 17,18 | C07C31/20 C07C29/03 B01J23/36 |
| A | WORLD PATENTS INDEX Week 6800, Derwent Publications Ltd., London, GB; AN 68-35166Q & JP-B-44 020 449 (ZAIDAN-HOJIN) * Zusammenfassung * | 1 | |
| A | US-A-3 646 130 (G.W. PARSHALL) * Anspruch 1 * | 1 | |
| A | EP-A-0 308 791 (DEGUSSA) * Seite 5, Zeile 12 * | 1 | RECHERCHIERTE SACHGEBIETE (Int. Cl.5 ) |
| D,A | US-A-3 518 285 (D.M. FENTON ET AL.) * Spalte 5, Zeile 16 - Spalte 5, Zeile 18 * | 1 | C07C B01J |
| D,A | DE-A-3 902 357 (HOECHST) * Seite 5, Zeile 31 * | 1 | |

-----

Der vorliegende Recherchenbericht wurde für alle Patentansprüche erstellt

| Recherchenort | Abschlußdatum der Recherche | Prüfer |
|---|---|---|
| BERLIN | 23 JULI 1992 | PROBERT C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)